(19) Europäisches Patentamt
European Patent Office
Office européen des brevets

(11) EP 2 681 186 B1

(12) **EUROPEAN PATENT SPECIFICATION**

(45) Date of publication and mention of the grant of the patent:
**11.05.2016 Bulletin 2016/19**

(21) Application number: **11706265.3**

(22) Date of filing: **03.03.2011**

(51) Int Cl.:
***C07C 211/42*** $^{(2006.01)}$ ***C07C 209/06*** $^{(2006.01)}$
***C07C 209/88*** $^{(2006.01)}$ ***C07B 57/00*** $^{(2006.01)}$

(86) International application number:
**PCT/EP2011/053211**

(87) International publication number:
**WO 2012/116752 (07.09.2012 Gazette 2012/36)**

(54) **PROCESS OF RESOLUTION OF 1-AMINOINDAN**

VERFAHREN ZUR AUFLÖSUNG VON 1-AMINOINDAN

PROCÉDÉ DE RÉSOLUTION DE 1-AMINOINDANE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**08.01.2014 Bulletin 2014/02**

(73) Proprietor: **Synthon BV**
**6545 CM Nijmegen (NL)**

(72) Inventor: **DYMÁCEK, Bohumil**
**67817 Blansko (CZ)**

(74) Representative: **Mendivil Gil, Maria Dolores**
**Synthon BV**
**IP Research**
**P.O. Box 7071**
**6503 GN Nijmegen (NL)**

(56) References cited:
**WO-A1-2009/147430**
**WO-A1-2010/049379**
**US-A1- 2007 112 217**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

## BACKGROUND OF THE INVENTION

The compound 1-aminoindan is an important chemical useful, e.g., as a starting material for making the pharmaceutically useful compound rasagiline, which is (R)-N-2-propynyl-1-indanamine of the formula (1)

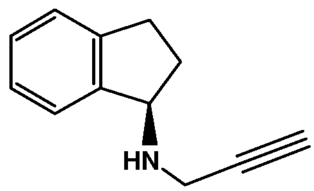

(1).

Rasagiline is a potent, selective, irreversible monoamine oxidase-type B (MAO-B) inhibitor for the treatment of Parkinson's disease, Alzheimer's disease and various types of dementia. The compound was disclosed in EP 436492. In the marketed pharmaceutical formulations, e.g. in tablets sold under the brand name AZILECT® (Teva Pharmaceutical Industries), the compound (1) is present as the methane sulfonate salt (i.e., rasagiline mesylate).

A known process of making rasagiline and its acid addition salts disclosed in EP 436492 (Teva) comprises reacting the R(-)-enantiomer of 1-aminoindan of formula (2) with propargyl bromide, propargyl chloride or propargylsulfonate ester in the presence of an organic or inorganic base in a suitable solvent and isolating the R(+)-enantiomer of the N-2-propynyl-1-indanamine by chromatography, distillation, selective extraction and, if desired, converting the free base obtained into an acid addition salt thereof.

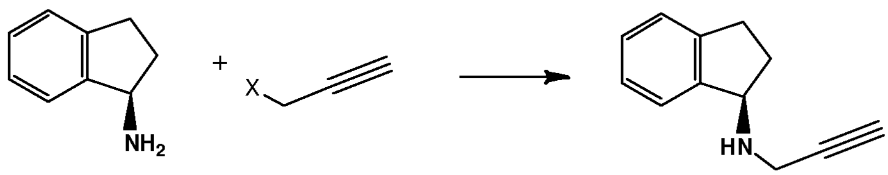

(2) (1)

Alternately, the starting material may be the racemic 1-aminoindan and the reaction mixture comprising the racemic rasagiline is treated with a suitable chiral acid to produce two diastereomeric salts, the desired (*R*)-N-2-propynyl-1-indanamine salt is separated by methods known per se, and, if desired, the free base is regenerated. Such process was disclosed in WO 95/11016.

From a commercial point of view, the approach starting from (*R*)-1-aminoindan is the most convenient way to make rasagiline.

Processes of making (*R*)-1-aminoindan are known in the art. An important group of these processes is based on a resolution of racemic 1-aminoindan as the racemate may be easily made.

EP 235590 discloses a process in which 1-aminoindan is resolved by fractional crystallization of its diastereomeric salts with D-N-acetyl-3,4-dimethoxyphenylalanine in methanol. This resolving agent is expensive and is not useful in an industrial process.

Ghislandi et al (Bull. Chim. Farm (1976),115: 489-500) discloses resolution of racemic 1-aminoindan by L-N-acetylleucine. As discussed in EP 235590, the yields and effectivity of the resolution are also very low.

In an article of Lawson and Rao (Biochemistry, vol. 19, pages 2133-2139 (1980)), racemic 1-aminoindan is resolved via fractional crystallization of its diastereomeric salts with L-malic acid in ethanol. However, the effectivity of this process is very low as it requires four times recrystallization.

WO 2009/147430 discloses a process in which 1-aminoindan is resolved by fractional crystallization of its diastereomeric salts with 2,3,4,6-di-O-isopropylidene-2-keto-L-gulonic acid in an organic solvent, preferably an alcohol.

US 5994408 discloses an alternate process for making (*R*)-1-aminoindan by resolution of N-benzyl-1-aminoindan using (*S*)-mandelic acid. Similarly, WO 02/068376 discloses a process involving preparation of N-benzyl-1-aminoindan, its resolution by L-tartaric acid and removal of the benzyl group by hydrogenolysis. This process is not advantageous as it involves multiple protection and deprotection steps.

In view of the above, there is still a need for an improved process for making (*R*)-1-aminoindan, particularly for a process, which is based on the resolution of racemic 1-aminoindan. Such process should be economical, easily realized

on industrial scale, with reasonable yields and purity of the product. Additionally, it would be desirable to find a suitable process for racemization of the undesired (*S*)-aminoindan, so that higher amounts of racemic 1-aminoindan may be economically useful.

## BRIEF DESCRIPTION OF THE PRESENT INVENTION

The present invention relates to the resolution of 1-aminoindan enantiomers by crystallization of diastereomeric salts thereof with naturally occurring chiral acids.

In particular, one aspect of the invention relates to a process of resolution of a mixture of (*R*)-and (*S*)-enantiomers of 1-aminoindan, said process comprising a step of fractional crystallizing one diastereomeric acid addition salt of 1-aminoindan from a solution containing a pair of diastereomeric acid addition salts of 1-aminoindan to form diastereomeric enriched solid and diastereomeric enriched solute, wherein the salt-forming acid is a chiral dicarboxylic acid selected from the group consisting of L(+)-aspartic acid, L(-)-malic acid and (2R,3R)-tartaric acid, and wherein the solvent for obtaining said solution is methanol. In an important aspect, the molar ratio of 1-aminoindan to the acid is about 1 : 1 in case of the two latter acids and/or about 2 : 1 in case of aspartic acid. The enriched (*R*)- or (*S*)-1-aminoindan enantiomer can be liberated as a free base from either the enriched solid or the enriched solute and may optionally be converted into an acid addition salt. The fractional crystallization can be repeated with or without first liberating the free base.

The aforementioned method leads to compounds selected from the group consisting of (*R*)-1-aminoindan L-aspartate, (*R*)-1-aminoindan L-hydrogenmalate and (*R*)-aminoindan hydrogen($2_R$, $3_R$)tartrate, respectively. These diastereomers are particularly useful in the fractional crystallization according to the present invention. Accordingly, these diastereomers are useful as starting materials for making the pharmaceutically useful compound rasagiline and/or acid addition salts thereof.

The (*S*)-enantiomer of 1-aminoindan can be racemized by reaction of said (*S*)-enantiomer with an alcoholate, e.g. with potassium tert-butoxide, in an organic solvent, which preferably is a dipolar aprotic solvent, more preferably dimethylsulfoxide.

## DETAILED DESCRIPTION OF THE PRESENT INVENTION

Not many ways to obtain enantiomerically pure *R*-1-aminoindan by resolution of a pair of diastereomeric salts of racemic 1-aminoindan with chiral acids are known in the literature. This is an indication that the resolution of this compound is not a trivial process. WO 96/21640 merely suggests using chiral organic acids to resolve 1-aminoindan. In every textbook on chiral separation the same suggestion can be found, but they also state that finding a suitable resolving agent for a given substrate is not trivial. Trial-and-error and practical experience is still the best methodology since no sound theoretical basis is available. If a resolution process is available for a certain compound, this method does not necessarily work for other substrates with closely related structures. It is apparent from the literature that only expensive N-acetyl-3-(3,4-dimethoxyphenyl)-D-alanine and/or 2,3,4,6-di-O-isopropylidene-2-keto-L-gulonic acid provide reasonably good results in case of 1-aminoindan resolution .

The present invention relates to the use of naturally occurring chiral organic acids as agents for resolution of racemic 1-aminoindan into enantiomers. In particular, it deals with the use of natural chiral dicarboxylic acids selected from L(+)-aspartic acid, L(-)-malic acid and (2R,3R)-tartaric acid for said purpose. While such use has been, at least in general, anticipated in the prior art, thorough study of the resolution conditions revealed that the nature of the resolution solvent and, also, the molar ratio of the resolution agent and crystallization conditions have an influence on the yield and effectivity of the resolution in an industrial process. Consequently, optimal resolution conditions for such industrial process were found.

Dicarboxylic acids, may, in essence, form two kinds of salts with 1-aminoindan: a monobasic (hydrogen-) salt of schematic formula (I) and a dibasic (hemi-) salt of schematic formula (II):

COOH-X-COOH . $NH_2$-indan (I)

COOH-X-COOH . $(NH_2\text{-indan})_2$ (II)

A mixture of enantiomers of 1-aminoindan forms a pair of diastereomers within each kind of salt, when the dicarboxylic acid is chiral and optically pure.

These salts may (or may not) differ in solubility both absolutely and relatively, i.e. while a pair of monobasic salts is soluble in a certain solvent, a similar pair of dibasic salts may be insoluble in the same solvent - and *vice versa*; furthermore, while the respective diastereomers within a pair of monobasic salts may substantially differ in solubility in a certain solvent, the solubility of the same diastereomers within a pair of dibasic salts may be almost identical in that solvent - and *vice versa*.

The present invention was successful in finding proper ratios between the 1-aminoindan and the corresponding optically pure chiral dicarboxylic acid yielding the proper pairs of isolatable diastereomeric salts, as well in finding the proper solvent, in which the pairs of salts exhibit significantly different solubility. As these acids are cheap and readily available and as the process exhibits a high resolution effectivity, the process of the present invention exhibits an economic advantage over the known resolution processes.

The process of resolving 1-aminoindan enantiomers comprises precipitating one diastereomeric dicarboxylate salt of 1-aminoindan from a solution in an aliphatic alcohol, advantageously in methanol, that contains a pair of diastereomeric dicarboxylate salts of 1-aminoindan to form a solid precipitate that is enriched with one diastereomer and a solute (or remaining mother liquor) that is enriched with the other diastereomer. Such a process is generally known as fractional crystallization. The "dicarboxylate salt", as used throughout the invention, is an abbreviation for a salt with a dicarboxylic acid selected from L(+)-aspartic acid, L(-)-malic acid and (2R,3R)-tartaric acid.

The substrate for the process of the invention ( = 1-aminoindan substrate) is a mixture of 1-aminoindan enantiomers. The mixture of (R) and (S) enantiomers can be equimolar (50:50) as in racemic 1-aminoindan or unequal. In some embodiments the amount of one enantiomer can be significantly greater than the amount of the other enantiomer, especially if the process is being applied to a 1-aminoindan substrate already partially resolved into enantiomers or to a substrate made by an optically specific method that has insufficient optical purity. The 1-aminoindan may be present either in the form of the free base or as an acid addition salt other than a salt with a chiral acid. In all these forms, the 1-aminoindan substrate may be applied in an isolated state, such as a crystalline or non-crystalline solid, a semisolid or liquid, or in a solution or as a product of a chemical reaction, i.e. as a raw product or reaction mixture obtained in the last step of the manufacturing process leading to it. It may be used either crude or purified by any suitable method, in any solvated or hydrated form.

The typical 1-aminoindan substrate for the process of optical resolution is the optically impure or racemic 1-aminoindan free base. Additionally, it is preferred that the 1-aminoindan free base be in chemically pure form, such as 80% or more pure, preferably 90% or more pure, and even 95% or more pure.

The racemic or otherwise optically impure 1-aminoindan substrate is converted into the diastereomeric salt pair by contacting the substrate in an aliphatic alcohol, typically in methanol, with the corresponding optically pure chiral dicarboxylic acid in a predetermined molar ratio. The proper molar ratio has basic importance as it has an influence both on the ability to crystallize and the crystallization yield of the corresponding salt and on the degree of enantiomeric enrichment of the crystallized solid salt. Thus, the proper molar ratio between 1-aminoindan and L-aspartic acid is approximately 2 : 1, while that between 1-aminoindan and L-malic acid or (2R,3R)-tartaric acid is approximately 1 : 1. Within the wording "approximately", the actual molar ratios may differ from the above numbers, but not more than 10% as higher difference may cause formation of various mixtures of salts, with an undesired influence on the yield and effectivity of the process.

In the process, a mixture of the 1-aminoindan substrate (free base or salt) with the solvent may be contacted with the corresponding amount of solid optically pure chiral acid, or a mixture of the chiral acid with the solvent may be contacted with solid 1-aminoindan, or both partners may be first combined with the solvent and then contacted. Under certain embodiments, a small (up to 10%) amount of water may be added to the solvent. Typically, the substrate and the acid are dissolved in the methanol solvent under heating, which includes reflux heating, but it is not strictly required. It is further not required that a complete solution is formed in this step, though it is preferred.

The salt reaction forms a pair of diastereomers in the solvent: one diastereomer resulting from the reaction of (*R*)-1-aminoindan with the optically pure chiral dicarboxylic acid and another resulting from the reaction of the (*S*)-1-aminoindan with such acid. Under the conditions of the present invention, the solution of the salt pair is subjected to fractional crystallization. The crystallization is "fractional" in that the conditions used allow for one of the diastereomers to be precipitated to a greater extent than the other. The crystallization of the solid precipitate may be spontaneous, or may be induced by changing the conditions of the solution, e.g. by cooling the mixture, adding a seed crystal, removal of a part of the solvent or by combination of these techniques. Preferred is to cool the obtained solution to a predetermined temperature, which is different for each of the three acids, and to allow crystallization at this temperature. The optimal crystallization temperature is of certain importance: at higher temperatures the yield is lower; at lower temperatures the degree of enrichment is lower.

The obtained solid salt is substantially enriched by one enantiomer of 1-aminoindan. As used herein "enrichment" means that the product contains more of one of the (*R*)- or (*S*)- 1-aminoindan or 1-aminoindan salt than the starting substrate or composition. Similarly, the mother liquor is likewise enriched by the other enantiomer, e.g. (*S*)-1-aminoindan, and thus is a diastereomeric enriched solute. It should be repeated that the ratio of diastereomers in the initial solution is not limited to racemic mixtures and includes solutions where one diastereomer is already contained in a greater amount than the other. In these latter cases, enrichment occurs so long as the solid (and solute) has a different ratio of one diastereomer to the other than in the starting solution.

More specifically, the diastereomeric salt of the desired (*R*) enantiomer of 1-aminoindan with L-aspartic or L-malic acid is less soluble in methanol than that of the (*S*) enantiomer and thus the (R)-enantiomer comprising salt

preferentially crystallizes out of the solution. When using L-aspartic acid as the resolution agent and methanol as the solvent, the typical concentration of the racemic 1-aminoindan is about 1 g per 50-75 ml of methanol and the typical crystallization temperature is about 50°C. When using L-malic acid as the resolution agent and methanol as the solvent, the typical concentration of the racemic 1-aminoindan is about 1 g per 10 ml of methanol and the typical crystallization temperature is about 35°C.

The precipitate may be separated from the reaction mixture by ordinary methods such as filtration or centrifugation.

To the contrary, the salt of the desired (*R*)-1-aminoindan enantiomer with (2*R*,3*R*)-tartaric acid is more soluble in methanol than that of the (*S*) enantiomer and it preferentially remains in the solution after separation of the solid. Thus, it is the mother liquor that is enriched by the desired (*R*)-enantiomer; it may be elaborated by various ways. For instance, the solvent may be evaporated or an antisolvent may be added to obtain the desired salt in solid state. Furthermore, the (R)-enantiomer may be obtained by a deep cooling of the mother liquor, e.g. to a temperature of about -20°C, where the salt of the (*R*)-enantiomer precipitates. Alternately, the (*R*)-enantiomer comprising salt is not isolated from the mother liquor in a solid state and the mother liquor is used as such in the next step of liberation of (*R*)-aminoindan from the salt.

The typical concentration of the racemic 1-aminoindan in this resolution process is about 1 g per 35-50 ml of methanol and the typical crystallization temperature of the (S)-enantiomer comprising salt is about 20°C.

The degree of enrichment resulting from the fractional crystallization according to the above disclosed process, expressed as optical purity calculated according to the formula

$$op\ [\%] = R / (R+S)\ \ x\ 100 \quad \text{or} \quad op\ [\%] = S / (R+S)\ \ x\ 100,$$

where R and S are the respective molar or mass amounts of the *R*- or *S*-enantiomers, is relatively high - typically at least 80% and in some embodiments at least 90%.

The optical purity can be yet increased by at least one re-crystallization of the diastereomeric enriched solid, preferably comprising the salt of the (*R*)-1-aminoindan with the optically pure chiral dicarboxylic acid, from the same or a different solvent (a recrystallization solvent); the recrystallization forms a further enriched solid and a recrystallization solute. The further enriched solid can be isolated by any known means as described above. Conversely, the recrystallization solute is advantageously combined with the initial solute to enhance the yield of the non-precipitating diastereomer.

In accordance with the above, preferred diastereomeric salt pairs include (*R*)-and (*S*)-1-aminoindan (hemi)L-aspartate, (*R*)- and (*S*)-1-aminoindan L-hydrogenmalate and (*R*)-and (*S*)-1-aminoindan hydrogen(2*R*,3*R*)tartrate. Each one of these diastereomers is a specific aspect of the present invention. The (*R*)-1-aminoindan containing diastereomer is particularly preferred as it may be used for making the desired (*R*)-aminoindan and, accordingly, rasagiline. However, the (*S*)-1-aminoindan containing diastereomer is useful as well as it may be subjected to a racemization reaction, which results in the formation of a next crop of racemic 1-aminoindan that may be re-used as a substrate for resolution into enantiomers.

The final optical purity of (*R*)-1-aminoindan in the prepared chiral salt is desired to be high, preferably is at least 90%, still more preferably at least 95%, and still more preferably at least 99% including 99.5% or more. In accordance with the above, such products may be obtained by the process of the present invention and thus form a specific aspect of the invention.

After the desired optical purity is obtained, the dicarboxylate salt substantially enriched by the desired (*R*)-enantiomer of 1-aminoindan, either the solid or the solute, may optionally be elaborated in the next step to liberate the so enriched (*R*)-1-aminoindan from the salt form as a free base. The liberation step essentially comprises treatment of the salt (in solid, suspended or dissolved state) with an organic or inorganic base. The base should be stronger than the 1-aminoindan, on the other hand it should not promote racemization.

The liberation step is advantageously performed in a solvent which at least partially dissolves the used salt and base. Generally, the liberation of the desired enantiomer of 1-aminoindan from the enriched carboxylate salt proceeds by contacting the salt with an equivalent of a suitable base, e.g., metal hydroxides, in water. The so formed free base of the enriched 1-aminoindan may be isolated by ordinary methods, e.g. by extraction with a water-immiscible organic solvent. Such extract may be used in a further reaction, e.g. in making rasagiline, as shown in more detail below, or the extraction solvent may be evaporated to provide the 1-aminoindan enantiomer in an isolated state. A suitable organic solvent for the extraction is a hydrocarbon, e.g. toluene.

Any conventional method applicable to the liberation of the 1-aminoindan base from a salt may be employed. In some embodiments, the process may be accompanied by a step of isolation of, and, if advantageous, the reprocessing of the resolution agent.

The formed enriched free base of the 1-aminoindan, especially the enriched (*R*) enantiomer, may be further converted into an acid addition salt with a suitable acid, by methods known per se. Preferred are salts that may be

obtained in a solid, particularly crystalline, form. Isolation of the (*R*)-aminoindan in a solid salt form is advantageous as such salt may be stored for prolonged time before its further use and may be easily handled in such further use. Examples of such salts are the hydrochloride, hydrobromide, acetate, fumarate, maleate, citrate or methane sulfonate salts.

The resulted free base and /or the salt of (*R*)-1-aminoindan preferably exhibit(s) an optical purity higher than 90% and preferably higher than 95%. Such product is "substantially optically pure". If the optical purity is lower than the desired one, the isolated product may be subjected to the new fractional crystallization process described above. The same or another chiral acid may be employed in the repeated process.

Similarly as above, a free base of 1-aminoindan enriched by the undesired (*S*)-enantiomer may be obtained. In an advantageous mode, such product may be subjected to racemization.

The racemization process is based on the reaction of the (*S*)-enantiomer of 1-aminoindan with an alcoholate, e.g. with potassium tert-butoxide, in an organic solvent, which preferably is a dipolar aprotic solvent, more preferably dimethylsulfoxide. The temperature of the reaction is typically 100°C or higher. After termination of the reaction, the reaction mixture is diluted with water, preferably with alkalized water, and the product is isolated by an extraction with a water-immiscible solvent, preferably with dichloromethane. The obtained racemic or substantially racemic 1-aminoindan may be subjected to the resolution process disclosed above. Thereby, the overall yield of the desired enantiomer is increased.

The optically pure or substantially pure (R)- enantiomer of 1-aminoindan and/or an acid addition salt thereof prepared by the process of the present invention, either in an isolated form or in a solution obtained in the process of making such enantiomer, is useful in making rasagiline. A typical process for making rasagiline comprises the N-alkylation of (*R*)-aminoindan with a propargyl halide, e.g. bromide, in the presence of a base. The base may be an inorganic base (a metal hydroxide or a carbonate) or an organic base (an amine, preferably a tertiary amine). If (*R*)-1-aminoindan is charged in a salt form, the amount of the base is present in such molar excess that also allows binding the acid ion by the base. Any suitable inert solvent may be used; in a preferred aspect, a biphasic solvent system comprising water and a water-immiscible organic solvent (advantageously a hydrocarbon , e.g. toluene) may be employed.

In a special aspect of the present invention, the starting material for such alkylation is the salt of (*R*)-1-aminoindan with the chiral dicarboxylic acid resulting from the process of resolution, i.e. with L-aspartic, L-malic or (2*R*,3*R*)-tartaric acid . In such arrangement, the above disclosed step of liberation of 1-aminoindan free base from the chiral salt is omitted, which makes the overall process shorter.

During the alkylation reaction, a certain amount of bis-alkylated side product is formed and a certain amount of starting 1-aminoindan remains unreacted. To remove the bis-alkylated impurity, the organic phase comprising the reaction products is extracted by acidified water at a pH of about 3.0, wherein only the product and unreacted 1-aminoindan dissolve in the aqueous layer. Rasagiline is then extracted back to toluene upon neutralization of the water layer to pH 6.0 with sodium hydroxide. Under these conditions, 1-aminoindan stays dissolved in the aqueous layer. From the toluene solution, rasagiline may be isolated by known means; it may be isolated as a base, e.g. by evaporation of the toluene, or in an acid addition salt form, e.g. as a hydrochloride or methane sulfonate, by adding the corresponding acid to the toluene solution of rasagiline. In an advantageous way, as disclosed in WO 2011/012140, the rasagiline is isolated as a hydrochloride salt by adding a solution of HCl in isopropanol to the toluene solution of the rasagiline base.

The present invention is more particularly described and explained by the following examples. It is to be understood, however, that the present invention is not limited to these examples and various changes and modifications may be made without departing from the scope of the present invention, said scope being solely defined and limited by the claims.

## EXAMPLES

### Example 1 Optical resolution of racemic 1-aminoindan by L(+)-aspartic acid (molar ratio 2 : 1.1)

0.55 g of L(+)-aspartic acid was stirred in 60 ml of boiling methanol upon forming a suspension. 1 g of racemic 1-aminoindan was dissolved in 5 ml of methanol and this solution was charged to the boiled suspension. The formed solution was cooled to 50°C, seeded and stirred at 50°C for 6 hours. Then the mixture was left spontaneously cooled with stirring to room temperature and stirred overnight. The precipitate was filtered and dried to obtain 0.54 g of (*R*)-1-aminoindan L-(+)-aspartate (66% of the theoretical value) with 91.9% optical purity (HPLC).

### Example 2 Optical resolution of racemic 1-aminoindan by L(-)-malic acid (molar ratio 1:1)

1g of racemic 1-aminoindan was dissolved in 5 ml of methanol. 1.06g of L(-)-malic acid was dissolved in 5 ml of methanol. The solution of 1-aminoindan was heated to 50°C and the solution of malic acid was added under stirring. The reaction mixture was cooled to 35°C, seeded and the formed suspension was held at 35°C for 1 hour. Then the mixture was cooled during 2 hours to 20°C and held at this temperature for 3 hours. The precipitate was filtered, washed

with 2 ml of methanol and dried to obtain 0.473 g of (*R*)-1-aminoindan hydrogen-L-(-)-malate (47% of the theoretical value). The optical purity was 99.75% (HPLC).

### Example 3 Optical resolution of racemic 1-aminoindan by (2*R*,3*R*)-tartaric acid (molar ratio 1 : 1)

11.83 g of (2*R*,3*R*)-tartaric acid was dissolved in 30 ml of boiling methanol and 1.35 g of water. 10 g of racemic 1-aminoindan was dissolved in 120 ml of methanol. Both solutions were combined together at reflux, diluted next with 250 ml of methanol and cooled to 20°C. After 30 minutes of stirring, the precipitate was filtered and dried to obtain 8.55 g of (*S*)-1-aminoindan hydrogen(2R,3R)tartrate. The optical purity was 84.6 % (HPLC).

The mother liquor was left overnight at 0-3°C. The formed precipitate was filtered off and dried to obtain 1.08g of (*R*)-1-aminoindan hydrogen(2*R*,3*R*)tartrate with an optical purity of 89.8%. The mother liquor was then stirred at -20°C for 3 hours, the formed suspension was filtered and dried to obtain 6.38 g of the second crop of (*R*)-1-aminoindan hydrogen(2*R*,3*R*)tartrate. The optical purity was 93.7% and the overall yield of the (R)-enantiomer was 66%.

### Example 4 Racemization of (*S*)-1-aminoindan

0.1 g of (*S*)-1-aminoindan was dissolved in 0.1 ml of dry dimethylsulfoxide and 0.01 g of potassium tert.butoxide was added. The reaction mixture was heated in argon atmosphere for 2 hours at 120°C. After cooling, the mixture was diluted with 0.5 ml of 15% aqueous NaOH and extracted with 2 x 0.5 ml of dichloromethane. The organic fraction was filtered with activated carbon, washed with 2 ml of ethanol and concentrated to dryness. The yield was 0.094 g (94% of the theoretical value), the chemical purity was 96% (HPLC), and the optical purity was 1.2 % ee (HPLC).

### Example 5 Conversion of (*R*)-1-aminoindan to rasagiline

A 500 ml round bottom flask was charged with 120 ml of water and 60.0 g of (*R*)-1-aminoindan hydrochloride was added with stirring. To this solution 5/8 of the initial volume of aqueous sodium hydroxide prepared from 29.7 g of NaOH and 60 ml of water were added. To the resulting emulsion, 47.3 g of propargyl bromide (80% solution in toluene) was added dropwise within 30 minutes. During this period the remaining 3/8 of aqueous sodium hydroxide were added in three portions. The stirring continued for 2 hours.

The reaction mixture was extracted with 2 x 50 ml of toluene. The combined organic layers were diluted with 50 ml of water and the pH was adjusted to 2.8-3.0 by 10% aqueous sulfuric acid. The aqueous layer was separated. The washing of the organic layer was repeated twice under the same conditions. The combined aqueous washings were mixed with 50 ml of toluene, the pH of the resulting emulsion was adjusted to 6.0- 6.2 by 15% aqueous NaOH and the organic layer was separated. The extraction of the aqueous layer was repeated twice under the same conditions.

The combined organic extracts were filtered and approximately 35 ml of 5-6 N hydrogen chloride in isopropanol was added dropwise within 30 minutes until the pH reached 2.0 - 3.0. The resulting suspension was refluxed for 30 minutes and then cooled to 20°C. The precipitate was filtered off, washed with 50 ml of isopropanol and dried at 60°C to afford 40.7 g of rasagiline hydrochloride. The optical purity was 100% by HPLC; the chemical purity was 99.86 % by HPLC.

## Claims

1. A process for resolution of a mixture of (*R*)-and (*S*)-enantiomers of 1-aminoindan, said process comprising a step of fractional crystallizing one diastereomeric acid addition salt of 1-aminoindan from a solution containing a pair of diastereomeric acid addition salts of 1-aminoindan to form a diastereomeric enriched solid and diastereomeric enriched solute, wherein the salt-forming acid is an optically pure chiral dicarboxylic acid selected from a group consisting of L(+)-aspartic acid, L(-)-malic acid and (2*R*,3*R*)-tartaric acid, and wherein the solvent for obtaining said solution is methanol.

2. The process according to claim 1, wherein the chiral dicarboxylic acid is L(+)-aspartic acid and the molar ratio of 1-aminoindan to the acid is about 2 : 1.

3. The process according to claim 1, wherein the chiral dicarboxylic acid is L(-)-malic acid and the molar ratio of 1-aminoindan to the acid is about 1 : 1.

4. The process according to claim 1, wherein the chiral dicarboxylic acid is (2*R*,3*R*)-tartaric acid and the molar ratio of 1-aminoindan to the acid is about 1 : 1.

5. The process according to claims 1-4 further comprising a step of liberating (R)- or (S)-1-aminoindan as a free base from either the enriched solid or the enriched solute.

6. The process according to claim 5 further comprising a step of converting the 1-aminoindan free base into an acid addition salt.

7. The process according to claims 1-6 wherein the 1-aminoindan and/or an acid addition salt thereof have an optical purity of higher than 90%.

8. A process for making rasagiline and/or acid addition salts thereof comprising the steps of:

- contacting a mixture of enantiomers of 1-aminoindan with an optically pure chiral dicarboxylic acid selected from a group consisting of L(+)-aspartic acid, L(-)-malic acid and (2*R*,3*R*)-tartaric acid in methanol and fractional crystallization of the salt of the (*R*)-1-aminoindan with the chiral dicarboxylic acid from the solution;
- optionally, at least one recrystallization of the salt of the (R)-1-aminoindan with the chiral dicarboxylic acid; and
- optionally, a step of liberating the (R)-1-aminoindan from the obtained salt of the (R)-1-aminoindan with the chiral dicarboxylic acid.
- converting enantiomerically pure (*R*)-1-aminoindan and/or its acid addition salt to rasagiline by a reaction thereof with propargylhalide, preferably propargyl bromide; and
- optionally, isolating rasagiline from the reaction mixture as a free base or as an acid addition salt.

9. The process according to claims 8, wherein the acid addition salt of rasagiline is hydrochloride or methanesulfonate.

## Patentansprüche

1. Verfahren zur Spaltung eines Gemisches von (*R*)- und (*S*)-Enantiomeren von 1-Aminoindan, wobei das Verfahren einen Schritt von fraktioniertem Kristallisieren eines diastereomeren Säureadditionssalzes von 1-Aminoindan aus einer Lösung umfasst, die ein Paar von diastereomeren Säureadditionssalzen von 1-Aminoindan enthält, um einen diastereomeren angereicherten Feststoff und ein diastereomeres angereichertes Solvat zu bilden, wobei die salzbildende Säure eine optisch reine chirale Dicarbonsäure ausgewählt aus einer Gruppe bestehend aus L(+)-Asparaginsäure, L(-)-Äpfelsäure und (2*R*, 3*R*)-Weinsäure ist, und wobei das Lösungsmittel zum Erhalten der Lösung Methanol ist.

2. Verfahren nach Anspruch 1, wobei die chirale Dicarbonsäure L(+)-Asparaginsäure ist, und das molare Verhältnis von 1-Aminoindan zur Säure etwa 2:1 beträgt.

3. Verfahren nach Anspruch 1, wobei die chirale Dicarbonsäure L(-)-Äpfelsäure ist, und das molare Verhältnis von 1-Aminoindan zur Säure etwa 1:1 beträgt.

4. Verfahren nach Anspruch 1, wobei die chirale Dicarbonsäure (2*R*, 3*R*)-Weinsäure ist, und das molare Verhältnis von 1-Aminoindan zur Säure etwa 1:1 beträgt.

5. Verfahren nach Ansprüchen 1-4, weiterhin umfassend einen Schritt des Freisetzens von (*R*)- oder (*S*)-1-Aminoindan als eine freie Base aus entweder dem angereicherten Feststoff oder dem angereicherten Solvat.

6. Verfahren nach Anspruch 5, weiterhin umfassend einen Schritt des Umwandelns der 1-Aminoindan-freien Base in ein Säureadditionssalz.

7. Verfahren nach Ansprüchen 1-6, wobei das 1-Aminoindan und/oder ein Säureadditionssalz davon eine optische Reinheit von höher als 90% aufweisen.

8. Verfahren zum Herstellen von Rasagilin und/oder Säureadditionssalzen davon, umfassend die Schritte:

- Inkontaktbringen eines Gemisches von Enantiomeren von 1-Aminoindan mit einer optisch reinen chiralen Dicarbonsäure ausgewählt aus einer Gruppe bestehend aus L(+)-Asparaginsäure, L(-)-Äpfelsäure und (2*R*, 3*R*)-Weinsäure in Methanol, und fraktioniertes Kristallisieren des Salzes des (*R*)-1-Aminoindans mit der chiralen Dicarbonsäure aus der Lösung;

- gegebenenfalls mindestens ein Umkristallisieren des Salzes des (R)-1-Aminoindans mit der chiralen Dicarbonsäure; und
- gegebenenfalls einen Schritt des Freisetzens des (R)-1-Aminoindans aus dem erhaltenen Salz des (R)-1-Aminoindans mit der chiralen Dicarbonsäure,
- Umwandeln des enantiomerisch reinen (*R*)-1-Aminoindans und/oder seines Säureadditionssalzes zu Rasagilin durch eine Umsetzung davon mit Propargylhalid, bevorzugt Propargylbromid; und
- gegebenfalls Isolieren von Rasagilin aus dem Reaktionsgemisch als eine freie Base oder als ein Säureadditionssalz.

**9.** Verfahren nach Anpruch 8, wobei das Säureadditionssalz von Rasagilin Hydrochlorid oder Methansulfonat ist.

## Revendications

**1.** Procédé de résolution d'un mélange des énantiomères (*R*) et (*S*) du 1-aminoindane, ledit procédé comprenant une étape de cristallisation fractionnée du sel d'addition d'un acide diastéréoisomère du 1-aminoindane d'une solution contenant une paire de sels d'addition diastéréoisomères du 1-aminoindane pour former un diastéréoisomère enrichi solide et un soluté diastéréoisomère enrichi, dans lequel l'acide formant le sel est un acide dicarboxylique chiral optiquement pur choisi dans le groupe comprenant l'acide L(+)aspartique, l'acide L(-) malique et l'acide (2*R*, 3*R*)-tartrique, et dans lequel le solvant pour obtenir ladite solution est le méthanol.

**2.** Procédé selon la revendication 1, dans lequel l'acide dicarboxylique chiral est l'acide L(+)-aspartique et le rapport molaire 1-aminoindane/acide est d'environ 2/1.

**3.** Procédé selon la revendication 1, dans lequel l'acide dicarboxylique chiral est l'acide L(-)-malique et le rapport molaire 1-aminoindane/acide est d'environ 1/1.

**4.** Procédé selon la revendication 1, dans lequel l'acide dicarboxylique chiral est l'acide (2*R*, 3*R*)-tartrique et le rapport molaire 1-aminoindane/acide est d'environ 1/1.

**5.** Procédé selon les revendications 1-4, comprenant en outre une étape de libération de (*R*)- ou (*S*)-1-aminoindane en tant que base libre à partir soit du solide enrichi soit du soluté enrichi.

**6.** Procédé selon la revendication 5, comprenant en outre une étape consistant à convertir la base libre du 1-aminoindane en un sel d'addition d'acide.

**7.** Procédé selon les revendications 1-6, dans lequel le 1-aminoindane et/ou un sel d'addition d'acide en dérivant présente une pureté optique supérieure à 90%.

**8.** Procédé de fabrication de rasagiline et/ou des sels d'addition d'acide en dérivant comprenant les étapes:

- une mise en contact d'un mélange d'énantiomères du 1-aminoindane avec un acide dicarboxylique chiral optiquement pur choisi dans le groupe comprenant l'acide L(+)-aspartique, l'acide L(-)malique et l'acide (2*R*, 3*R*)-tartrique dans du méthanol et une cristallisation fractionnée du sel de (*R*)-1-aminoindane avec l'acide dicarboxylique chiral de la solution;
- éventuellement, au moins une recristallisation du sel de (*R*)-1-aminoindane avec l'acide dicarboxylique chiral; et
- éventuellement une étape de libération de (*R*)-1-aminoindane du sel de la (*R*) -1-aminoindane avec l'acide dicarboxylique chiral obtenu;
- la conversion de l'énantiomère pur de (*R*)-1-aminoindane et/ou de son sel d'addition d'acide en rasagiline par une réaction de celui-ci avec un halogénure de propargyl, de préférence le bromure de propargyle; et
- éventuellement, l'isolement de la rasagiline du mélange réactionnel sous forme d'une base libre ou d'un sel d'addition d'acide.

**9.** Procédé selon la revendication 8, dans lequel le sel d'addition d'acide de la rasagiline est le chlorhydrate ou un méthanesulfonate.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 436492 A
- WO 9511016 A
- EP 235590 A
- WO 2009147430 A
- US 5994408 A
- WO 02068376 A
- WO 9621640 A
- WO 2011012140 A


### Non-patent literature cited in the description


- **GHISLANDI et al.** *Bull. Chim. Farm,* 1976, vol. 115, 489-500
- **LAWSON ; RAO.** *Biochemistry,* 1980, vol. 19, 2133-2139